# EUROPÄISCHE PATENTSCHRIFT

(11) **EP 0 802 790 B1**
(45) Veröffentlichungstag und Bekanntmachung des Hinweises auf die Patenterteilung: **06.04.2005**
(21) Anmeldenummer: 96900309.4
(22) Anmeldetag: 10.01.1996
(51) Int. Cl.: A61K 9/70, A61K 31/42

(54) **TRANSDERMALES THERAPEUTISCHES SYSTEM ZUR VERABREICHUNG VON (S)-3-METHYL-5-(1-METHYL-2-PYRROLIDENYL)-ISOXAZOL ODER EINEM SEINER PHARMAZEUTISCH AKZEPTABLEN SALZE**
TRANSDERMAL THERAPEUTIC SYSTEM FOR DISPENSING (S)-3-METHYL-5-(1-METHYL-2-PYRROLIDENYL)-ISOXAZOLE OR ONE OF ITS PHARMACEUTICALLY ACCEPTABLE SALTS
SYSTEME THERAPEUTIQUE TRANSDERMIQUE D'ADMINISTRATION DE (S)-3-METHYL-5-(1-METHYL-2-PYRROLIDENYL)-ISOXAZOLE OU D'UN DE SES SELS ACCEPTABLES SUR LE PLAN PHARMACEUTIQUE

(30) Priorität: 14.01.1995 DE 19501022
(43) Veröffentlichungstag der Anmeldung: 29.10.1997
(73) Patentinhaber: ABBOTT LABORATORIES, Abbott Park, Illinois 60064-3500 (US)
(72) Erfinder: FRANKE, Hanshermann, D-56566 Neuwied (DE); HOFFMANN, Gerd, D-56626 Andernach (DE); KINDEL, Heinrich, D-56581 Ehlscheidt (DE); ARNERIC, Stephen, Peter, Lindenhurst, IL 60046 (US); SULLIVAN, James, Patrick, Abbott Laboratories, Abbott Park, IL 60064-3500 (US); CANNON, John, Burns, Abbott Laboratories, Abbott Park, IL 60064-3500 (US)
(74) Vertreter: Flaccus, Rolf-Dieter, Dr.
(86) Internationale Anmeldenummer: PCT/EP1996/000074
(87) Internationale Veröffentlichungsnummer: WO 1996/021434

(56) Entgegenhaltungen:
- WO-A-94/25025
- WO-A-94/26257
- WO-A-95/15754
- SOCIETY FOR NEUROSCIENCE ABSTRACTS, Bd. 20, Nr. 1-2, 13.November 1994 Seite 174 XP 000564993 KANG ET AL 'transdermal abt-418: pharmacokinetic and pharmacodynamic properties of a cholinergic channel activator'
- DRUG DEVELOPMENT AND INDUSTRIAL PHARMACY, Bd. 13, Nr. 4&5, 1987 Seiten 589-651, Y. W. CHIEN 'Development of transdermal drug delivery sytems'

## Beschreibung

Die Erfindung betrifft eine Darreichungsform für die Verabreichung von (s)-3-Methyl-5-(1-methyl-2-pyrrolidinyl)-isoxazol oder einem seiner pharmazeutisch akzeptablen Salze und ein Verfahren zu ihrer Herstellung.

Der Wirkstoff (s)-3-Methyl-5-(1-methyl-2-pyrrolidinyl)-isoxazol zeichnet sich durch eine hochspezifische agonistische Wirkung auf das cholinergische System, im besonderen auf die nikotinischen Rezeptoren aus. Die Applikation dieses Wirkstoffs scheint aufgrund neuerer Untersuchungen auf dem Gebiet der Alzheimer-Therapie die kognitiven Fähigkeiten von Erkrankten deutlich zu verbessern. Hierfür ist jedoch die regelmäßige und mengenmäßig konstante Verabreichung des Wirkstoffes ein wichtiger Faktor, um gleichbleibend wirksame Blutspiegel über längere Zeiträume sicherzustellen. Bisher ist die ausschließlich orale Verabreichung des Wirkstoffs bekannt. Die orale Verabreichung von Medikamenten bei Demenz-Patienten stellt jedoch unzumutbar hohe Anforderungen an das Krankenpersonal, da der Patient durch seine Krankheit nicht in der Lage ist, die Einnahme selbstständig zu kontrollieren.

Der Erfindung liegt die Aufgabe zugrunde, zur Erzielung einer hochspezifischen agonistischen Wirkung auf das cholinergische System von Demenz-Patienten eine Darreichungsform für die Verabreichung des Wirkstoffs (s)-3-Methyl-5-(1-methyl-2-pyrrolidinyl)-isoxazol anzugeben, durch welche unter Ablösung der bisher erforderlichen regelmäßigen oralen Verabreichung in kurzen Intervallen ein konstant wirksamer Blutspiegel erzielbar ist, und wodurch zum Wohl des Patienten die bisher als Nachteil empfundene Belastung und Aufmerksamkeit des Pflegepersonals erleichtert wird.

Kang, C. H. et al., Society for Neuroscience Abstracts, Vol. 20, 1994, Seite 174, beschreibt die pharmakokinetischen Eigenschaften von (s)-3-Methyl-5-(1-methyl-2-pyrrolidinyl)-isoxazol bei Ratten und zeigt auf, daß bei den Versuchstieren die Wirkung dieses Wirkstoffs mit dessen Plasmaspiegel korreliert. Bei den beschriebenen Versuchen wurde ein für die Wirkung ausreichender Plamaspiegel mit Hilfe einer speziellen wirkstoffhaltigen Kammer erreicht.

Zur Lösung der Aufgabe wird mit der Erfindung eine für die Verabreichung von (s)-3-Methyl-5-(1-methyl-2-pyrrolidinyl)-isoxazol oder eines seiner pharmazeutisch akzeptablen Salze neue Darreichungsform angegeben, welche darin besteht, daß diese in Pflasterform als transdermales therapeutisches System (TTS) vorliegt.

WO 95/15754 offenbart ein transdermales therapeutisches System zur Applikation von (s)-3-Methyl-5-(1-methyl-2-pyrrolidinyl)-isoxazol, bei dem die wirkstoffhaltig Haftklebeschicht aus einem Acrylat-Copolymer besteht oder zwei ebenfalls aus einem Acrylat-copolymer bestehende, wirkstoffhaltige Haftklebeschichten durch eine Membran voneinander getrennt sind.

Gegenstand der Erfindung ist daher ein TTS zur Verabreichung von (s)-3-Methyl-5-(1-methyl-2-pyrrolidinyl)-isoxazol oder einem seiner pharmazeutisch geeigneten Salze, welches in Pflasterform vorliegt und eine geeignete Rückschicht, ein damit verbundenes Wirkstoffreservoir, bei Abwesenheit anderer Steuermechanismen eine die Abgabe des Wirkstoffs steuernde Membran, eine Haftklebeeinrichtung zur Befestigung des Systems auf der Haut und im Bedarfsfall eine vor der Applikation des Systems wieder ablösbare Schutzschicht umfaßt wobei das Wirkstoffreservoir einen beutelartigen oder schichtförmigen Aufbau mit mindestens einer Polymerschicht aufweist, und das Material der Polymerschicht aus den Gruppen der Polyisobutylene, Polyterpene, Ethylen-Vinylacetat-Copolymere, der Blockpolymere, der Synthese kautschuke oder Heißschmelzklebern ausgewählt ist.

Dabei kann die geeignete Rückschicht sowohl aus einem flexiblen als auch nicht flexiblen wirkstoffundurchlässigen Material bestehen. Substanzen, die zu ihrer Herstellung verwendet werden können, sind Polymer- oder Metallfolien, wie Aluminiumfolien, die allein oder mit einem polymeren Substrat beschichtet angewandt werden können. Darüber hinaus können auch textile Flächengewebe verwendet werden, wenn das Reservoir aufgrund ihrer physikalischen Beschaffenheit nicht durch sie hindurchtreten kann oder das Hindurchtreten durch eine entsprechende Schicht verhindert wird.

Das Wirkstoffreservoir weist entweder einen schichtförmigen oder einen beutelartigen Aufbau auf.

Das schichtförmig aufgebaute Reservoir kann wahlweise aus einer polymeren Matrix und dem reinen Wirkstoff oder einer geeigneten Wirkstoffzubereitung bestehen, wobei.das System als solches durch die Polymereigenschaften zusammengehalten wird. Das Reservoir besteht aus einem Grundpolymer und üblichen Zusätzen. Die Auswahl der polymeren Matrix und des Grundpolymers richtet sich nach den physikochemischen Eigenschaften des Wirkstoffs einerseits und der angestrebten Freisetzung aus dem System andererseits. Grundsätzlich kommen alle Polymere in Frage, die bei der Herstellung von Haftklebern eingesetzt werden und welche mit dem Wirkstoff verträglich sind. Für die erfindungsgemäße Darreichungsform zu nennen sind hier: Kautschuk, kautschukähnliche Homo-, Co- oder Blockpolymere. Besonders bevorzugt sind solche auf der Basis von Styrol und 1,3-Dienen, Polyisobutylenen und Polyterpenen. Von den Blockpolymeren auf Basis von Styrol und 1,3-Dienen werden bevorzugt lineare Styrol-Isopren-Blockpolymere eingesetzt.

Die Art und Menge der möglichen Zusätze hängt von der eingesetzten polymeren Matrix und dem wirkstoff ab. Entsprechend ihrer Funktion lassen sie sich in folgende Gruppen einteilen: Weichmacher, Klebrigmacher, Stabilisatoren, Trägerstoffe, diffusions- und penetrationsregulierende Zusätze oder Füllstoffe. Die hierfür in Frage kommenden physiologisch unbedenklichen Substanzen sind dem pharmazeutischen Fachmann bekannt.

Ein beutelförmiges Reservoirsystem kann aus dem reinen Wirkstoff oder aus einer hochviskosen oder halbfesten oder thixotropen Wirkstoffzubereitung bestehen. Besonders geeignet sind ölige oder wäßrige Gele, die in einen aus geeigneten Folien- oder Membranmaterialien hergestellten Beutel eindosiert werden. Die Herstellung der Wirkstoffzubereitung kann dabei auf der Basis von lipophilen oder hydrophilen Grundlagen erfolgen. Die der Haut abgewandte Beutelrückseite muß dabei wirkstoffundurchlässig und die der Haut zugewandte Seite wirkstoffdurchlässig sein. Vorzugsweise kann eine wirkstoffdurchlässige Membran die Steuerung der Wirkstofffreisetzung übernehmen.

Bei Abwesenheit anderer Steuermechanismen kann das Reservoir mit einer die Abgabe des Wirkstoffs steuernden Membran und einer Haftklebeeinrichtung zur Befestigung des Systems auf der Haut verbunden werden. Einen besonderen Fall stellt ein auf der Haut nicht ausreichend klebendes Reservoir dar, welches mit einer speziellen Haftklebeschicht verbunden wird, die einen ständigen Kontakt des Systems zur Haut sicherstellt. Für die Haftklebeeinrichtung kommen prinzipiell die gleichen Materialien wie für die polymere Matrix des Reservoirs in Frage.

Die im Bedarfsfall vor der Applikation abzulösende Schutzschicht kann aus den gleichen Materialien bestehen, wie sie für die Rückschicht des Systems Verwendung finden, vorausgesetzt, daß sie z.B. durch eine Silikonisierung ablösbar gemacht wurden. Es können aber auch andere ablösbare Schutzschichten wie Polytetrafluorethylen-behandeltes Papier, Cellophan, Polyvinylchlorid, Polyvinylidenchlorid und ähnliche verwendet werden. Wird das erfindungsgemäße Laminat vor dem Aufbringen der Schutzschicht in therapiegerechte Formate (Pflaster) aufgeteilt, so können die dann aufzubringenden Schutzschichtformate ein überstehendes Ende aufweisen, mit dessen Hilfe sie leichter von dem Pflaster abziehbar sind.

Entsprechend dem schichtartigen oder beutelartigen Aufbau der zuvor beschriebenen transdermalen Systeme werden zwei unterschiedliche Verfahren zur Herstellung beschrieben:
a: Der Wirkstoff wird mit den Bestandteilen des Wirkstoffreservoirs gegebenenfalls in Lösung oder unter Erwärmen und Abkühlen homogen vermischt und auf die wirkstoffdurchlässige Deckschicht oder ein darauf befindliches textiles Flächengewebe aufgebracht, worauf gegebenenfalls das oder die Lösemittel entfernt werden. Je nachdem, ob die Reservoirschicht selbstklebend ist oder nicht, wird zwischen dem Reservoir und der im Bedarfsfalle ablösbaren Schutzschicht eine Haftklebeeinrichtung zur dauernden Befestigung auf der Haut eingebracht.
b: Der flüssige Wirkstoff oder die flüssige oder halbfeste oder thixotrope Wirkstoffzubereitung wird innerhalb einer geeigneten Apparatur auf eine wirkstoffdurchlässige Membran dosiert und mit einem geeigneten Folienmaterial weitgehend blasenfrei abgedeckt, woraufhin Membran und Folienmaterial zu einem Beutel dicht versiegelt werden. Wahlweise kann die Dosierung auch in textiles Flächengewebe erfolgen, welches zusätzlich auch eine Stützfunktion erfüllen kann. Auch das Eindosieren in bereits vorgeformte Beutel ist möglich. Der wirkstoffgefüllte Reservoirbeutel wird ferner mit einer Haftklebeeinrichtung, die auch mehrschichtig aufgebaut und mit einer Steuerfunktion zur Wirkstofffreisetzung versehen
sein kann, verbunden und anschließend mit einer im Bedarfsfall ablösbaren Schutzschicht bedeckt.

## Patentansprüche

1. Darreichungsform für die Verabreichung von (s)-3-Methyl-5-(1-methyl-2-pyrrolidinyl)-isoxazol oder einem seiner pharmazeutisch akzeptablen Salze, wobei die Darreichungsform als transdermales therapeutisches System in Form eines Pflasters vorliegt und eine geeignete Rückschicht, ein darauf kaschiertes Wirkstoffreservoir, eine Haftklebeeinrichtung zur Befestigung des Systems auf der Haut und im Bedarfsfall eine vor der Applikation des Systems ablösbare Schutzschicht umfaßt, wobei das Wirkstoffreservoir einen beutelartigen oder schichtförmigen Aufbau mit mindestens einer Polymerschicht aufweist, und das Material der Polymerschicht ausgewählt ist aus den Gruppen der Polyisobutylene, der Polyterpene, der Ethylen-Vinylacetat-Copolymere, der Blockpolymere, der Synthesekautschuke oder Heißschmelzkleber.

2. Darreichungsform nach Anspruch 1, **dadurch gekennzeichnet, daß** die Rückschicht des Systems feuchtigkeits- und wirkstoffundurchlässig ist.

3. Darreichungsform nach Anspruch 1, **dadurch gekennzeichnet, daß** die Rückschicht wirkstoffdurchlässig ist.

4. Darreichungsform nach einem der Ansprüche 1 bis 3, **dadurch gekennzeichnet, daß** die Darreichungsform bei Abwesenheit anderer Steuermechanismen eine die Abgabe von Wirkstoff steuernde Membran aufweist.

5. Darreichungsform nach einem der Ansprüche 1 bis 4, **dadurch gekennzeichnet, daß** die Polymerschicht Ester des hydrierten Kolophoniums, insbesondere Methyl- oder Glycerinester des hydrierten Kolophoniums enthält.

6. Darreichungsform nach einem oder mehreren der Ansprüche 1 - 5, **dadurch gekennzeichnet, daß** es einen Weichmacher in einer Konzentration von 0 - 30 %, vorzugsweise von 5 - 20 %, bezogen auf das Gesamt-Matrixgewicht, enthält.

7. Darreichungsform nach Anspruch 6, **dadurch gekennzeichnet, daß** der Weichmacher mindestens einen Bestandteil aus den Gruppen der Kohlenwasserstoffe, Alkohole, Carbonsäuren und ihrer Derivate, Ether, Ester oder Amine enthält.

8. Darreichungsform nach Anspruch 6, **dadurch gekennzeichnet, daß** der Weichmacher ein natürliches oder partialsynthetisches Triglycerid oder eine Mischung dieser Triglyceride in einem Konzentrationsbereich von 0 - 30 %, vorzugsweise im Bereich 5 - 20 %, bezogen auf das Gesamt-Matrixgewicht, enthält.

9. Darreichungsform nach einem oder mehreren der vorhergehenden Ansprüche, **dadurch gekennzeichnet, daß** es einen Permeationsbeschleuniger in einem Konzentrationsbereich von 0 - 30 %, vorzugsweise im Bereich 5 - 20 %, bezogen auf das Gesamt-Matrixgewicht, enthält.

10. Darreichungsform nach Anspruch 9, **dadurch gekennzeichnet, daß** der Permeationsbeschleuniger mindestens einen Bestandteil aus den Gruppen der Kohlenwasserstoffe, Alkohole, Carbonsäuren und ihrer Derivate, Ether, Ester oder Amine enthält.

11. Darreichungsform nach Anspruch 9, **dadurch gekennzeichnet, daß** der Permeationsbeschleuniger mindestens einen Bestandteil aus der Gruppe der Mono-, Di- oder Sesquiterpene enthält.

12. Darreichungsform nach Anspruch 9, **dadurch gekennzeichnet, daß** der Permeationsbeschleuniger mindestens einen Bestandteil aus der Gruppe der Polyoxyethylen-Derivate enthält.

13. Darreichungsform nach Anspruch 9, **dadurch gekennzeichnet, daß** der Permeationsbeschleuniger einen Bestandteil aus der Gruppe der stickstoffhaltigen Heterozyklen und ihrer Derivate enthält.

14. Darreichungsform nach Anspruch 9, **dadurch gekennzeichnet, daß** der Permeationsbeschleuniger einen Bestandteil aus der Gruppe der Pyrrolidon-Derivate enthält.

15. Darreichungsform nach einem oder mehreren der vorhergehenden Ansprüche, **dadurch gekennzeichnet, daß** es ein beutelförmiges, mit einer flüssigen oder halbfesten oder thixotropen Matrix gefülltes Wirkstoffreservoir besitzt.

16. Darreichungsform nach Anspruch 15, **dadurch gekennzeichnet, daß** die Grundlage des halbfesten oder thixotropen Wirkstoffreservoirs ein Gelbildner ist.

17. Verfahren zur Herstellung der Darreichungsform nach einem oder mehreren der vorherigen Ansprüche, **dadurch gekennzeichnet, daß** (s)-3-Methyl-5-(1-methyl-2-pyrrolidinyl)-isoxazol oder dessen pharmazeutisch akzeptables Salz in Lösung auf ein textiles Flächenmaterial auf einer vorgefertigten und gegebenenfalls mit einer Schutzschicht versehenen Matrix aufgebracht wird, wonach Matrix und Wirkstoffdepot mit einer Deckschicht versehen werden.

18. Verfahren nach Anspruch 17, **dadurch gekennzeichnet, daß** Bestandteile eines Schmelzklebers vor Zugabe des Wirkstoffs unter Erwärmen auf bevorzugt 110 - 170 °C bis zum Erhalt einer homogenen Schmelze von 70 bis 100 °C eingearbeitet werden.

19. Verfahren nach Anspruch 17 oder 18, **dadurch gekennzeichnet, daß** die homogene wirkstoffhaltige Schmelzklebermasse durch Extrusion, Gießen, Walzenauftrag, Rakelauftrag oder durch ein Druckverfahren aufgebracht wird.

## Claims

1. An administration form for the administration of (s)-3-methy(-5-(1-methyl-2-pyrrolidinyl)-isoxazole or one of its pharmaceutically acceptable salts, said administration form being present as a transdermal therapeutic system in the form of a patch and comprising a suitable backing layer, an active substance reservoir laminated thereon, a pressure-sensitive adhesive device to attach the system to the skin, and, if required, a protective layer removable prior to application of the system, and said active substance reservoir having a bag-type or a layered structure with at least one polymer layer, and the material of the polymer layer being selected from the groups of the polyisobutylenes, polyterpenes, ethylene-vinyl-acetate copolymers, block polymers, synthetic rubbers, or hot-melt adhesives.

2. The administration form according to claim 1 **characterized in that** the backing layer of the system is impermeable to moisture and active substance.

3. The administration form according to claim 1 **characterized in that** the backing layer is permeable to the active substance.

4. The administration from according to any one of claims 1 to 3, **characterized in that** in the absence of other control mechanisms said administration form comprises a membrane controlling the release of active substance.

5. The administration form according to any one of claims 1 to 4 **characterized in that** the polymer layer comprises esters of hydrogenated colophony, in particular methyl or glycerol esters of hydrogenated colophony.

6. The administration form according to one or several of claims 1 - 5 **characterized in that** it comprises a plasticizer in a concentration of 0 - 30%, preferably 5 - 20%, relative to the total weight of the matrix.

7. The administration form according to claim 6 **characterized in that** the plasticizer comprises at least one component selected from the groups of hydrocarbons, alcohols, carboxylic acids and their derivatives, ethers, esters, or amines.

8. The administration form according to claim 6 **characterized in that** the plasticizer comprises a natural or partially synthetic triglyceride or a mixture of these triglycerides in a concentration ranging from 0 - 30%, preferably in the range of 5 - 20%, relative to the total weight of the matrix.

9. The administration form according to one or several of the preceding claims **characterized in that** it comprises a permeation enhancer in a concentration ranging from 0 - 30%, preferably in the range of 5 - 20%, relative to the total weight of the matrix.

10. The administration form according to claim 9 **characterized in that** the permeation enhancer comprises at least one component selected from the groups of hydrocarbons, alcohols, carboxylic acids and their derivatives, ethers, esters, or amines.

11. The administration form according to claim 9 **characterized in that** the permeation enhancer comprises at least one component selected from the group consisting of mono-, di- or sesquiterpenes.

12. The administration form according to claim 9 **characterized in that** the permeation enhancer comprises at least one component selected from the group consisting of polyoxyethylene derivatives.

13. The administration form according to claim 9 **characterized in that** the permeation enhancer comprises a component selected from the group consisting of nitrogen-containing heterocycles and their derivatives.

14. The administration form according to claim 9 **characterized in that** the permeation enhancer comprises a component selected from the group consisting of pyrrolidone derivatives.

15. The administration form according to one or several of the preceding claims **characterized in that** it has a bag-type active substance reservoir filled with a liquid or semisolid or thixotropic matrix.

16. The administration form according to claim 15 **characterized in that** a gel former is the base of the semisolid or thixotropic active substance reservoir.

17. A process for the production of the administration form according to one or several of the preceding claims **characterized in that** (s)-3-methyl-5- (1-methyl-2- pyrrolidinyl)- isoxazole or its pharmaceutically acceptable salt is applied in solution on a textile fabric provided on a prefabricated matrix which is optionally provided with a protective layer, whereupon matrix and active substance depot are provided with a cover layer.

18. The process according to claim 17 **characterized in that** components of a hot-melt adhesive are incorporated, prior to the addition of the active substance, under heating to preferably 110 - 170°C until a homogeneous melt of 70 to 100°C is obtained.

19. The process according to claim 17 or 18 **characterized in that** the homogeneous, active-substance-containing hot-melt adhesive mass is applied by means of extrusion, casting, roller coating, knife coating, or by a printing process.

## Revendications

1. Forme pharmaceutique pour l'administration du (s)-3-méthyl-5-(1-méthyl-2-pyrrolidinyl)-isoxazole ou d'un de ses sels pharmaceutiquement acceptables, la forme pharmaceutique étant présente à titre de système thérapeutique transdermique sous la forme d'un emplâtre et comprenant une couche dorsale appropriée, un réservoir pour substance active contrecollé sur la première citée, un dispositif auto-adhésif pour la fixation du système sur la peau et, en cas de nécessité, une couche de protection détachable avant l'application du système, le réservoir pour substance active présentant une structure analogue à celle d'une poche ou une structure stratifiée comprenant au moins une couche polymère, la matière de la couche polymère étant choisie parmi les groupes des polyisobutylènes, des polyterpènes, des copolymères d'éthylène-acétate de vinyle, des polymères séquencés, des caoutchoucs de synthèse ou des colles thermofusibles.

2. Forme pharmaceutique selon la revendication 1, **caractérisée en ce que** la couche dorsale du système est imperméable à l'humidité et aux substances actives.

3. Forme pharmaceutique selon la revendication 1, **caractérisée en ce que** la couche dorsale est perméable aux substances actives.

4. Forme pharmaceutique selon l'une quelconque des revendications 1 à 3, **caractérisée en ce que** la forme pharmaceutique, en l'absence d'autres mécanismes de commande, présente une membrane qui commande la libération des substances actives.

5. Forme pharmaceutique selon l'une quelconque des revendications 1 à 4, **caractérisée en ce que** la couche polymère contient des esters de colophane hydrogénée, en particulier l'ester méthylique ou l'ester glycérolique de colophane hydrogénée.

6. Forme pharmaceutique selon une ou plusieurs des revendications 1 à 5, **caractérisée en ce qu'**elle contient un plastifiant en une concentration de 0 à 30 %, de préférence de 5 à 20 %, rapportés au poids total de la matrice.

7. Forme pharmaceutique selon la revendication 6, **caractérisée en ce que** le plastifiant contient au moins un constituant choisi parmi les groupes des hydrocarbures, des alcools, des acides carboxyliques et de leurs dérivés, des éthers, des esters ou des amines.

8. Forme pharmaceutique selon la revendication 6, **caractérisée en ce que** le plastifiant contient un triglycéride naturel ou partiellement synthétique ou encore un mélange de ces triglycérides dans une plage de concentration de 0 à 30 %, de préférence dans la plage de 5 à 20 %, rapportés au poids total de la matrice.

9. Forme pharmaceutique selon une ou plusieurs des revendications précédentes, **caractérisée en ce qu'**elle contient un accélérateur de perméation dans une plage de concentration de 0 à 30 %, de préférence dans la plage de 5 à 20 %, rapportés au poids total de la matrice.

10. Forme pharmaceutique selon la revendication 9, **caractérisée en ce que** l'accélérateur de perméation contient au moins un constituant choisi parmi les groupes des hydrocarbures, des alcools, des acides carboxyliques et de leurs dérivés, des éthers, des esters ou des amines.

11. Forme pharmaceutique selon la revendication 9, **caractérisée en ce que** l'accélérateur de perméation contient au moins un constituant choisi parmi le groupe des monoterpènes, des diterpènes ou des sesquiterpènes.

12. Forme pharmaceutique selon la revendication 9, **caractérisée en ce que** l'accélérateur de perméation contient au moins un constituant choisi parmi le groupe des dérivés du polyoxyéthylène.

13. Forme pharmaceutique selon la revendication 9, **caractérisée en ce que** l'accélérateur de perméation contient un constituant choisi parmi le groupe des hétérocycles azotés et de leurs dérivés.

14. Forme pharmaceutique selon la revendication 9, **caractérisée en ce que** l'accélérateur de perméation contient un constituant choisi parmi le groupe des dérivés de la pyrrolidone.

15. Forme pharmaceutique selon une ou plusieurs des revendications précédentes, **caractérisée en ce qu'**elle possède la forme d'une poche comprenant un réservoir pour substance active rempli avec une matrice liquide ou semi-solide ou thixotrope.

16. Forme pharmaceutique selon la revendication 15, **caractérisée en ce que** la base du réservoir semi-solide ou thixotrope pour substances actives est un agent gélifiant.

17. Procédé pour la préparation de la forme pharmaceutique selon une ou plusieurs des revendications précédentes, **caractérisé en ce qu'**on applique du (s)-3-méthyl-5-(1-méthyl-2-pyrrolidinyl)-isoxazole ou son sel pharmaceutiquement acceptable en solution sur un produit plat textile sur une matrice préfabriquée et, le cas échéant, munie d'une couche de protection, avant de munir la matrice et le dépôt de substances actives d'une couche de recouvrement.

18. Procédé selon la revendication 17, **caractérisé en ce qu'**on incorpore des constituants d'une colle thermofusible avant l'addition de la substance active, en chauffant de préférence à une température de 110 à 170 °C jusqu'à ce que l'on obtienne une masse fondue homogène de 70 à 100 °C.

19. Procédé selon la revendication 17 ou 18, **caractérisé en ce qu'**on applique la matière thermofusible homogène contenant les substances actives, par extrusion, par versage, par application au rouleau, par application à la racle ou via un procédé d'impression.
